# FASCICULE DE BREVET EUROPEEN

(11) **EP 2 585 504 B1**
(45) Date de publication et mention de la délivrance du brevet: **06.08.2014**
(21) Numéro de dépôt: 11728359.8
(22) Date de dépôt: 06.06.2011
(51) Int. Cl.: C08F 220/18, C08F 220/28, C08F 220/56, C08F 226/10, A61K 8/81, A61K 47/32, A61Q 5/00, A61Q 19/00, C08F 283/06

(54) **NOUVEAU POLYMERE EPAISSISSANT DE PHASES GRASSES SANS MONOMERE IONIQUE**
NEUES VERDICKUNGSPOLYMER OHNE IONISCHEN MONOMERE FÜR ÖLPHASEN
NOVEL THICKENING POLYMER FOR OIL PHASES FREE OF IONIC MONOMERS

(30) Priorité: 22.06.2010 FR 1054933
(43) Date de publication de la demande: 01.05.2013
(73) Titulaire: Societe D Exploitation De Produits Pour Les Industries Chimiques Seppic, 75007 Paris (FR)
(72) Inventeur: BRAUN, Olivier, F-81100 Castres (FR); OLLAGNIER, Jean-Noêl, F-81100 Castres (FR)
(74) Mandataire: Conan, Philippe Claude
(86) Numéro de dépôt international: PCT/FR2011/051275
(87) Numéro de publication internationale: WO 2011/161349

(56) Documents cités:
- WO-A1-2006/002936
- WO-A2-2006/064151
- FR-A1- 2 910 899

## Description

La présente demande de brevet concerne de nouveaux composés épaississant les phases huile, leur procédé de préparation et leur application en tant qu'épaississants et/ou émulsionnants pour des produits de soins de la peau, des cheveux et du cuir chevelu notamment les émulsions cosmétiques, dermocosmétiques, dermopharmaceutiques ou pharmaceutiques eau-dans huile.

Des polymères épaississants synthétiques, se présentant sous forme de latex inverses, sont décrits comme pouvant être utilisés dans la fabrication de compositions topiques, dans les demandes de brevet européen publiées sous les numéros EP 0 716 594, EP 1 047 716, EP 1 056 805 et EP 0 503 853.

Cependant, la plupart de ces épaississants ne sont pas capables d'épaissir les phases huile.

La demande de brevet européen publiée sous le numéro EP 0 406 042 divulgue des compositions cosmétiques sous forme d'émulsions eau dans huile contenant comme agent épaississant un polymère ayant une faible proportion de motifs à groupement ioniques tel que par exemple le copolymère de N-dodécyl acrylamide et d'acide acrylamido-2 méthyl-2 propanesulfonique (AMPS) dans une proportion pondérale 96,5 / 3,5 ou le copolymère de N-tertiobutyl acrylamide et d'acide acrylamido-2 méthyl-2 propane sulfonique dans une proportion pondérale 97,9 / 2,1. De tels copolymères sont d'un usage délicat dans l'industrie cosmétique, car ils doivent être neutralisés avant utilisation, et que leur solubilisation dans les huiles nécessite souvent l'emploi d'un cosolvant. De plus leur aptitude à épaissir les huiles est faible.

C'est la raison pour laquelle les seuls composés connus aujourd'hui possédant cette propriété, sont les copolymères commercialisés sous le nom INTELIMER qui sont copolymères hydrophobes ayant de longues chaînes alkyle pendantes qui cristallisent à froid pour former des clusters provoquant l'épaississement du milieu. De tels polymères du type copolymères d'acrylate d'alkyle et d'acide (méth)acrylique sont décrits dans les brevets américains publiés sous les numéros US 7,101,928 B1 et US 5,736,125. Néanmoins l'utilisation de ces produits n'est pas simple car il faut dissoudre le polymère à chaud dans l'huile puis procéder à un refroidissement pour provoquer la cristallisation des chaînes. De plus, ils sont par nature sensibles à la température et les compositions épaissies avec ce type de polymère sont délicates à commercialiser dans les pays chauds.

C'est pourquoi la demanderesse a cherché à développer de nouveaux polymères capables d'épaissir les phases huiles et organiques qui n'aient pas les inconvénients exposés ci-dessus.

L'invention a donc pour objet des copolymères à base de méthacrylate de stéaryle ainsi qu'un procédé pour leur préparation en phase apolaire.

Ce copolymère caractérisé en ce qu'il comporte pour 100% molaire :
a) plus de 70% molaire et jusqu'à 99,9% molaire d'unités monomériques hydrophobes (A) issues du méthacrylate de stéaryle ; et
b) de 0,1% molaire à 30% molaire d'unités monomériques (B) issues d'au moins un monomère d'un composé de formule (I) : 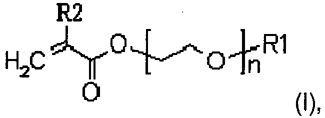
dans laquelle R1 représente un radical alkyle, linéaire ou ramifié, comportant de 12 à 22 atomes de carbone, R2 représente un atome d'hydrogène ou un radical méthyle et n un nombre entier supérieur ou égal à 0 et inférieur ou égal 30, étant entendu que ledit composé de formule (I) n'est pas le méthacrylate de stéaryle.

Dans la définition précédente, par copolymère, on désigne les polymères d'unités monomériques issues d'au moins deux monomères de structure chimique différente. Le terme copolymères inclut donc aussi les terpolymères, les tétrapolymères et les polymères d'unités monomériques issues de plus de quatre monomères de structure chimique différente.

Dans la formule (I) telle que définie précédemment, lorsque le radical R1 représente un radical alkyle linéaire comprenant de 12 à 22 atomes de carbone, il s'agit plus particulièrement d'un radical choisi parmi les radicaux dodécyle, tétradécyle, hexadécyle, octadécyle, eicosyle ou docosyle.

Dans la formule (I) telle que définie précédemment, lorsque le radical R1 représente un radical alkyle ramifié comprenant de 12 à 22 atomes de carbone, il peut s'agir du radical de formule :

CH(CₙH₂ₙ₊₁)(CₘH₂ₘ₊₁)-CH₂-

dans lequel m est un nombre entier égal compris entre 1 et 4, n est un nombre entier compris entre 9 et 16 ; ou du radical de formule :

CH(CₚH₂ₚ₊₁)₂ -CH₂-

dans lequel p est un nombre entier égal compris entre 5 et 10 ;

Selon un aspect particulier de la présente invention, dans la formule (I) telle que définie précédemment, le radical R1 représente un radical alkyle linéaire choisi parmi les radicaux dodécyle ou docosanyle.

Selon un aspect particulier de la présente invention, dans la formule (I) telle que définie précédemment, le radical R2 représente le radical méthyle.

Selon un aspect particulier de la présente invention, dans la formule (I) telle que définie précédemment, n est supérieur à zéro ; il est plus particulièrement supérieur ou égal à quatre et inférieur ou égal à vingt-cinq.

Selon un aspect particulier de la présente invention, l'unité monomérique (B) est issue du méthacrylate de béhènyle pentacosaéthoxylé, composé qui correspond à la formule (I) telle que définie précédemment, dans laquelle R1 représente le radical docosanyle, R2 représente le radical méthyle et n est égal à 25.

Selon un autre aspect particulier de la présente invention, l'unité monomérique (B) est issue du méthacrylate de lauryle tétraéthoxylé, composé qui correspond à la formule (I) telle que définie précédemment, dans laquelle R1 représente le radical dodécyle, R2 représente le radical méthyle et n est égal à 4.

Selon un autre aspect particulier, dans le copolymère tel que défini précédemment, le ratio molaire (A)/(B) est compris entre 80/20 et 95/5.

L'invention a plus particulièrement pour objet les copolymères suivants :
- Copolymère de méthacrylate de stéaryle et de méthacrylate de béhènyle pentacosaéthoxylé [ratio molaire (A)/(B) = 95/5],
- Copolymère de méthacrylate de stéaryle et de méthacrylate de béhènyle pentacosaéthoxylé [ratio molaire (A)/(B)= 90/10],
- Copolymère de méthacrylate de stéaryle et de méthacrylate de béhènyle pentacosaéthoxylé [ratio molaire (A)/(B) = 85/15], ou du :
- Copolymère de méthacrylate de stéaryle et de méthacrylate de béhènyle pentacosaéthoxylé [ratio molaire (A)/(B) = 80/20].

L'invention a aussi pour objet un copolymère tel que défini précédemment, caractérisé en ce qu'il comporte en outre, pour 100% molaire, de 0,1% molaire à 30% molaire d'unités monomériques (C) issues d'au moins un monomère neutre choisi parmi l'acrylamide, le méthacrylamide, le N,N-diméthyl acrylamide, le N,N-diéthyl acrylamide, le N-isopropyl acrylamide, l'acrylate de (2-hydroxy éthyle), l'acrylate de (2,3-dihydroxy propyle), le méthacrylate de (2-hydroxy éthyle) le méthacrylate, de (2,3-dihydroxy propyle) ou la N-vinyl pyrrolidone et plus particulièrement, le N,N-diméthyl acrylamide ou le N,N-diéthyl acrylamide.

Selon un mode particulier, l'invention a pour objet un copolymère tel que défini précédemment comportant pour 100% molaire :
a) de 80% molaire à 95% molaire d'unités monomériques hydrophobes (A) ;
b) de 2,5% molaire à 10% molaire d'unités monomériques (B) ; et
c) de 2,5% molaire à 10% molaire d'unités monomériques (C) ;
et plus particulièrement les copolymères suivants :
- Terpolymère de méthacrylate de stéaryle, de méthacrylate de béhènyle pentacosaéthoxylé et de N,N-diméthyl acrylamide [ratio molaire (A)/(B) /(C) = 87/5/8] ;
- Terpolymère de méthacrylate de stéaryle, de méthacrylate de béhènyle pentacosaéthoxylé et de N,N-diméthyl acrylamide [ratio molaire (A)/(B)/(C) = 80/5/15] ;
- Terpolymère de méthacrylate de stéaryle, de méthacrylate de béhènyle pentacosaéthoxylé et de N,N-diméthyl acrylamide [ratio molaire (A)/(B) /(C) = 85/10/5] ;
- Terpolymère de méthacrylate de stéaryle, de méthacrylate de béhènyle pentacosaéthoxylé et de N,N-diméthyl acrylamide [ratio molaire (A)/(B)/(C) = 80/10/10] ;
- Terpolymère de méthacrylate de stéaryle, de méthacrylate de béhènyle pentacosaéthoxylé et de N,N-diméthyl acrylamide [ratio molaire (A)/(B)/(C) = 90/5/5] ; ou du,
- Terpolymère de méthacrylate de stéaryle, de méthacrylate de béhènyle pentacosaéthoxylé et de N,N-diméthyl acrylamide [ratio molaire (A)/(B)/(C) = 95/2,5/2,5)].

L'invention a aussi pour objet un procédé de préparation du copolymère tel que défini précédemment, comprenant :
- Une étape a) de préparation d'une phase organique par mélange dans les proportions molaires souhaitées, d'au moins une huile avec le méthacrylate de stéaryle, le monomère de formule (I) et, le cas échéant, le monomère neutre ;
- Une étape b) de polymérisation amorcée par introduction dans le mélange résultant de l'étape a), d'un amorceur thermique pour obtenir une solution dans l'huile du copolymère attendu ; et si désiré
- Une étape c) d'élimination de l'huile de la solution de copolymère obtenue à l'étape b).

Dans le procédé tel que défini précédemment, l'huile mise en oeuvre peut être une huile minérale ou une huile d'origine végétale ou d'un mélange d'huiles minérales et/ou d'origine végétales. L'huile ou le mélange d'huiles mis en oeuvre dans le procédé objet de la présente invention doit être liquide à température ambiante et plus généralement dans l'intervalle dans l'intervalle de température compris entre 15°C et 45°C ou pour certaines utilisations entre 4°C et 45°C.

Par huile minérale, on désigne notamment dans le procédé tel que défini précédemment, les paraffines les isoparaffines ou les cycloparaffines présentant à température ambiante, une densité entre 0,7 et 0,9 et un point d'ébullition supérieur à environ 150°C, telle que par exemple :
- L'ISOPAR™ H, l'ISOPAR™ G ou l'ISOPAR™ M,
- Le MARCOL™ 52, une huile commerciale répondant à la définition des huiles de vaseline du Codex français et qui est une huile blanche minérale conforme aux réglementations FDA 21 CFR 172.878 et CFR 178.3620 (a) elle est inscrite à la Pharmacopée des USA, US XXIII (1995) et à la Pharmacopée européenne (1993) ;

- L'isohexadécane, identifié dans Chemical Abstract par le numéro RN = 4390-04-9 qui est un mélange d'isoparaffines en C12, C16, C20 contenant au moins 97% d'isoparaffines en C16 parmi lesquelles le constituant principal est le 2, 2, 4, 4, 6, 8, 8-heptaméthyl nonane (RN = 4390-04-9) ;
- L'isododécane.

Par huile végétale on désigne notamment dans le procédé tel que défini précédemment, une huile végétale de type ester ou triglycéride, comme le coco caprylate caprate, par exemple le DUB™ 810C fourni par la société Dubois, ou encore l'huile de Jojoba.

L'étape a) du procédé tel que défini précédemment, est réalisée sous agitation jusqu'à obtenir une dissolution complète des monomères mis en oeuvre.

L'étape b) du procédé tel que défini précédemment, est typiquement réalisée à une température d'environ 80°C, et laissée se dérouler jusqu'à polymérisation complète.

Par amorceur thermique on désigne dans l'étape c) du procédé tel que défini précédemment, amorceur thermique, notamment l'azo bis(isobutyronitrile) (AIBN) ou le peroxyde de lauroyle.

L'étape c) d'élimination de l'huile optionnellement mise en oeuvre dans la procédé tel que défini précédemment, est généralement réalisée par précipitation préalable du copolymère formé à l'issue de l'étape b) avec un solvant de précipitation approprié tel que par exemple l'acétone, puis filtration du précipité. On aussi procéder à l'élimination de l'huile par atomisation de la solution obtenue à l'étape b).

L'invention a aussi pour objet la solution de copolymère directement obtenue à l'issue de l'étape b) du procédé tel que défini ci-dessus.

L'invention a aussi pour objet l'utilisation du copolymère tel que défini précédemment ou de la solution de polymère telle que définies ci-dessus, comme agent modificateur de la rhéologie dans une composition topique cosmétique, dermopharmaceutique ou pharmaceutique et plus particulièrement l'utilisation telle que définie ci-dessus, dans laquelle la composition topique cosmétique, dermopharmaceutique ou pharmaceutique est une émulsion de type eau dans huile.

Une composition topique selon l'invention, destinée à être appliquée sur la peau, sur le cheveu, sur le cuir chevelu ou sur les muqueuses de l'homme ou de l'animal, peut consister en une émulsion topique comprenant au moins une phase aqueuse et au moins une phase huile. Cette émulsion topique est plus particulièrement du type eau dans huile. La phase huile de l'émulsion topique peut consister en un mélange d'une ou plusieurs huiles.

Une composition topique selon l'invention peut être destinée à une utilisation cosmétique ou être utilisée pour préparer un médicament destiné au traitement des maladies de la peau, du cuir chevelu et des muqueuses. Dans ce dernier cas, la composition topique comporte alors un principe actif qui peut par exemple consister en un agent anti-inflammatoire, un agent myorelaxant, un agent antifongique ou un agent antibactérien.

Lorsque la composition topique est utilisée en tant que composition cosmétique destinée à être appliquée sur la peau, sur le cuir chevelu ou les muqueuses, elle peut ou non comporter un principe actif, par exemple un agent hydratant, un agent bronzant, un filtre solaire, un agent antirides, un agent à visée amincissante, un agent anti-radicalaire, un agent anti-acnéique ou un agent antifongique.

La composition topique peut en outre comporter des composés classiquement compris dans ce type de compositions, par exemple des parfums, des conservateurs, des antioxydants, des colorants, des émollients ou des tensioactifs.

L'invention a aussi pour objet un procédé pour modifier la rhéologie d'une composition topique cosmétique, dermopharmaceutique ou pharmaceutique comprenant une phase grasse, caractérisé en ce que l'on introduit dans ladite phase grasse une quantité efficace du copolymère tel que défini précédemment ou de la solution de polymère telle que définie précédemment.

L'invention a enfin pour objet une composition topique cosmétique, dermopharmaceutique ou pharmaceutique et plus particulièrement une émulsion eau dans huile selon invention comporte habituellement pour 100% de sa masse totale entre 0,1% massique et 10% massique et plus particulièrement entre 1% massique et 5 % massique du copolymère tel que défini ci-dessus.

Les exemples qui suivent ont pour but d'illustrer la présente invention.

### Exemple 1 : Copolymères de méthacrylate de stéaryle et de méthacrylate de béhènyle pentacosaéthoxylé dans l'ISOPAR™ H.

### Préparation

a) - On introduit d'abord 56,2 g d'ISOPAR™ H dans un réacteur. On verse ensuite progressivement et sous agitation en maintenant la température autour de 30°C, 35,5 g de méthacrylate de stéaryle, 8,3 g de méthacrylate de béhènyle pentacosaéthoxylé.
b) - Après dégazage sous azote pendant environ une demi-heure, la température est portée à 80°C puis la polymérisation est initiée par ajout de 0,5% molaire d'azobis(isobutyronitrile). On laisse la polymérisation se dérouler pendant 7 heures et l'on obtient le copolymère attendu en solution dans l'huile.

On obtient une solution dans l'huile du copolymère de méthacrylate de stéaryle et de méthacrylate de béhènyle pentacosaéthoxylé (ratio molaire A / B = 95 / 5) dénommé ci après composé 1A.

En faisant varier la proportion molaire en monomères indiqués, on prépare de la même manière les solutions dans l'huile des copolymères suivants :
- Copolymère de méthacrylate de stéaryle et de méthacrylate de béhènyle pentacosaéthoxylé (ratio molaire A / B = 90 / 10) dénommé ci après composé 1 B ;
- Copolymère de méthacrylate de stéaryle et de méthacrylate de béhènyle pentacosaéthoxylé (ratio molaire A / B = 85 / 15) dénommé ci après composé 1C ;
- Copolymère de méthacrylate de stéaryle et de méthacrylate de béhènyle pentacosaéthoxylé (ratio molaire A / B = 80 / 20) dénommé ci après composé 1 D.

### Evaluation des propriétés

On ajoute à chacune des solutions de copolymères 1A à 1 D, du MARCOL™ 52 de façon à obtenir une solution à 5% massique de copolymère, puis on complète avec 1% massique d'eau pour 100% de phase huile.

Chacun des échantillons de phase grasse est ensuite placé 30 secondes au bain marie à 80°C puis maintenu sous agitation à 500 tours par minute pendant 3 heures environ. Les observations en termes d'aspect visuel des solutions des copolymères et de consistance des gels obtenus, sont consignées dans le tableau ci-dessous.

| | Copolymère | | | |
|---|---|---|---|---|
| | 1A | 1B | 1C | 1D |
| Aspect de la solution dans l'huile | Limpide | Limpide | Limpide | Limpide |
| Consistance du gel final | *** | ** | ** | ** |

| | | | | |
|---|---|---|---|---|
| Echelle de viscosité : * : Insuffisamment visqueux, ** : Visqueux, *** : Plus visqueux | | | | |

### Exemple 2 : Terpolymères de méthacrylate de stéaryle, de méthacrylate de béhènyle pentacosaéthoxylé et de N,N-diméthyl acrylamide dans l'ISOPAR™ H.

### Préparation

a) - On introduit d'abord 56,2 g d'ISOPAR™ H dans un réacteur. On verse ensuite progressivement et sous agitation en maintenant la température autour de 30°C, 34,16 g de méthacrylate de stéaryle, 8,72 g de méthacrylate de béhènyle pentacosaéthoxylé et 0,792 g de N,N-diméthyl acrylamide.
b) - Après dégazage sous azote pendant environ une demi-heure, la température est portée à 80°C puis la polymérisation est initiée par ajout de 0,5% molaire d'azobis(isobutyronitrile). On laisse la polymérisation se dérouler pendant 7 heures et l'on obtient le copolymère attendu en solution dans l'huile.

On obtient une solution dans l'huile du terpolymère de méthacrylate de stéaryle, de méthacrylate de béhènyle pentacosaéthoxylé et de N,N-diméthyl acrylamide (ratio molaire A / B = 87/5/8) dénommé ci après composé 2A.

En faisant varier la proportion molaire en monomères indiqués, on prépare de la même manière les solutions dans l'huile des copolymères suivants :
- Terpolymère de méthacrylate de stéaryle, de méthacrylate de béhènyle pentacosaéthoxylé et de N,N-diméthyl acrylamide (ratio molaire A / B / C = 80/5/15) dénommé ci après composé 2B.
- Terpolymère de méthacrylate de stéaryle, de méthacrylate de béhènyle pentacosaéthoxylé et de N,N-diméthyl acrylamide (ratio molaire A / B / C = 85/10/5) dénommé ci après composé 2C.
- Terpolymère de méthacrylate de stéaryle, de méthacrylate de béhènyle pentacosaéthoxylé et de N,N-diméthyl acrylamide (ratio molaire A / B / C = 80/10/10) dénommé ci après composé 2D.
- Terpolymère de méthacrylate de stéaryle, de méthacrylate de béhènyle pentacosaéthoxylé et de N,N-diméthyl acrylamide (ratio molaire A / B / C = 90/5/5) dénommé ci après composé 2E.
- Terpolymère de méthacrylate de stéaryle, de méthacrylate de béhènyle pentacosaéthoxylé et de N,N-diméthyl acrylamide (ratio molaire A / B / C = 95/2,5/2,5) dénommé ci après composé 2F.

### Evaluation des propriétés

On ajoute à chacune des solutions de copolymères 2A à 2F, du MARCOL™ 52 de façon à obtenir une solution à 5% massique de copolymère, puis on complète avec 1% massique d'eau pour 100% de phase huile.

Chacun des échantillons de phase grasse est ensuite placé 30 secondes au bain marie à 80°C puis maintenu sous agitation à 500 tours par minute pendant 3 heures environ. Les observations en termes d'aspect visuel des solutions des copolymères et de consistance des gels obtenus, sont consignées dans le tableau ci-dessous.

| | Copolymère | | | | | |
|---|---|---|---|---|---|---|
| | 2A | 2B | 2C | 2D | 2E | 2F |
| Aspect de la solution dans l'huile | Limpide | Limpide | Limpide | Limpide | Limpide | Limpide |
| Consistance du gel final | **** | **** | ** | ** | **** | ** |

| | | | | | | |
|---|---|---|---|---|---|---|
| Echelle de viscosité : * : Insuffisamment visqueux, ** : Visqueux, *** : Plus visqueux | | | | | | |

### Exemple comparatif : Copolymère de méthacrylate de stéaryle et de N,N-diméthyl acrylamide dans l'ISOPAR™ H.

### Préparation

a) - On introduit d'abord 56,2 g d'ISOPAR™ H dans un réacteur. On verse ensuite progressivement et sous agitation en maintenant la température autour de 30°C, 41,6 g de méthacrylate de stéaryle et 1,1 g de N,N-diméthyl acrylamide.
b) - Après dégazage sous azote pendant environ une demi-heure, la température est portée à 80°C puis la polymérisation est initiée par ajout de 0,5% molaire d'azobis(isobutyronitrile). On laisse la polymérisation se dérouler pendant 7 heures et l'on obtient le copolymère attendu en solution dans l'huile.

On obtient une solution dans l'huile du copolymère de méthacrylate de stéaryle et de N,N-diméthyl acrylamide (ratio molaire A / B = 85 / 15) dénommé ci après composé C.

### Evaluation des propriétés

On ajoute à la solution de copolymère C, du MARCOL™ 52 de façon à obtenir une solution à 5% massique de copolymère, puis on complète avec 1% massique d'eau pour 100% de phase huile.

L'échantillon de phase grasse est ensuite placé 30 secondes au bain marie à 80°C puis maintenu sous agitation à 500 tours par minute pendant 3 heures environ. Les observations en termes d'aspect visuel de la solution de copolymère et de consistance du gel obtenus, sont consignées dans le tableau ci-dessous.

| | Copolymère C |
|---|---|
| Aspect de la solution dans l'huile | Limpide |
| Consistance du gel final | * |

| | |
|---|---|
| Echelle de viscosité : * : Insuffisamment visqueux | |

Ces essais mettent en évidence que les copolymères selon l'invention, sont de bons agents épaississants d'huile en ce qu'ils modifient leur rhéologie en nécessitant le minimum d'eau, contrairement aux polymères de l'état de la technique, ce qui permet d'obtenir aisément des gels clairs.

La comparaison avec le copolymère C de l'exemple comparatif, met aussi en évidence que la présence dans les copolymères selon l'invention, d'unités monomériques issues du composé de formule (I), est essentielle.

## Revendications

1. Copolymère **caractérisé en ce qu'**il comporte pour 100% molaire :
a) plus de 70% molaire et jusqu'à 99,9% molaire d'unités monomériques hydrophobes (A) issues du méthacrylate de stéaryle ; et
b) de 0,1% molaire à 30% molaire d'unités monomériques (B) issues d'au moins un monomère d'un composé de formule (I) : 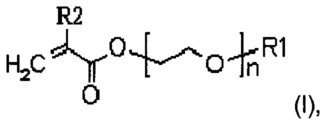 dans laquelle R1 représente un radical alkyle, linéaire ou ramifié, comportant de 12 à 22 atomes de carbone, R2 représente un atome d'hydrogène ou un radical méthyle et n un nombre entier supérieur ou égal à 0 et inférieur ou égal 30, étant entendu que ledit composé de formule (I) n'est pas le méthacrylate de stéaryle.

2. Copolymère tel que défini à la revendication 1, pour lequel dans la formule (I), le radical R1 représente un radical alkyle linéaire choisi parmi les radicaux dodécyle ou docosanyle.

3. Copolymère tel que défini à l'une quelconque des revendications 1 ou 2, pour lequel dans la formule (I), n est supérieur à zéro et plus particulièrement, n est supérieur ou égal à quatre et inférieur ou égal à vingt-cinq.

4. Copolymère tel que défini à la revendication 3 **caractérisé en ce qu'**il s'agit du copolymère de méthacrylate de stéaryle et de méthacrylate de béhènyle pentacosaéthoxylé ou du copolymère de méthacrylate de stéaryle et d'acrylate de lauryle tétraéthoxylé.

5. Copolymère tel que défini à l'une quelconque des revendications 1 à 4, dans lequel le ratio molaire (A)/(B) est compris entre 80/20 et 95/5.

6. Copolymère tel que défini à l'une quelconque des revendications 4 ou 5, **caractérisé en ce qu'**il s'agit du :
- Copolymère de méthacrylate de stéaryle et de méthacrylate de béhènyle pentacosaéthoxylé [ratio molaire (A)/(B) = 95/5],
- Copolymère de méthacrylate de stéaryle et de méthacrylate de béhènyle pentacosaéthoxylé [ratio molaire (A)/(B)= 90/10],
- Copolymère de méthacrylate de stéaryle et de méthacrylate de béhènyle pentacosaéthoxylé [ratio molaire (A)/(B) = 85/15], ou du :
- Copolymère de méthacrylate de stéaryle et de méthacrylate de béhènyle pentacosaéthoxylé [ratio molaire (A)/(B) = 80/20].

7. Copolymère tel que défini à l'une des revendications 1 à 6, **caractérisé en ce qu'**il comporte en outre, pour 100% molaire, de 0,1% molaire à 30% molaire d'unités monomériques (C) issues d'au moins un monomère neutre choisi parmi l'acrylamide, le méthacrylamide, le N,N-diméthyl acrylamide, le N,N-diéthyl acrylamide, le N-isopropyl acrylamide, l'acrylate de (2-hydroxy éthyle), l'acrylate de (2,3-dihydroxy propyle), le méthacrylate de (2-hydroxy éthyle) le méthacrylate, de (2,3-dihydroxy propyle) ou la N-vinyl pyrrolidone.

8. Copolymère tel que défini à la revendication 9, dans lequel ladite unité monomérique (C), est issue du N,N-diméthyl acrylamide ou du N,N-diéthyl acrylamide.

9. Copolymère tel que défini à l'une quelconque des revendications 7 ou 8, comportant pour 100% molaire :
a) de 80% molaire à 95% molaire d'unités monomériques hydrophobes (A) ;
b) de 2,5% molaire à 10% molaire d'unités monomériques (B) ; et
c) de 2,5% molaire à 10% molaire d'unités monomériques (C).

10. Copolymère tel que défini à l'une quelconque des revendications 8 ou 9, **caractérisé en ce qu'**il s'agit du :
- Terpolymère de méthacrylate de stéaryle, de méthacrylate de béhènyle pentacosaéthoxylé et de N,N-diméthyl acrylamide [ratio molaire (A)/(B) /(C) = 87/5/8] ;
- Terpolymère de méthacrylate de stéaryle, de méthacrylate de béhènyle pentacosaéthoxylé et de N,N-diméthyl acrylamide [ratio molaire (A)/(B)/(C) = 80/5/15] ;
- Terpolymère de méthacrylate de stéaryle, de méthacrylate de béhènyle pentacosaéthoxylé et de N,N-diméthyl acrylamide [ratio molaire (A)/(B) /(C) = 85/10/5] ;
- Terpolymère de méthacrylate de stéaryle, de méthacrylate de béhènyle pentacosaéthoxylé et de N,N-diméthyl acrylamide [ratio molaire (A)/(B)/(C) = 80/10/10] ;
- Terpolymère de méthacrylate de stéaryle, de méthacrylate de béhènyle pentacosaéthoxylé et de N,N-diméthyl acrylamide [ratio molaire (A)/(B)/(C) = 90/5/5] ; ou du,
- Terpolymère de méthacrylate de stéaryle, de méthacrylate de béhènyle pentacosaéthoxylé et de N,N-diméthyl acrylamide [ratio molaire (A)/(B)/(C) = 95/2,5/2,5)].

11. Procédé de préparation du copolymère tel que défini à l'une quelconque des revendications 1 à 10, comprenant :
- Une étape a) de préparation d'une phase organique par mélange dans les proportions molaires souhaitées, d'au moins une huile avec le méthacrylate de stéaryle, le monomère de formule (I) et, le cas échéant, le monomère neutre ;
- Une étape b) de polymérisation amorcée par introduction dans le mélange résultant de l'étape a), d'un amorceur thermique pour obtenir une solution dans l'huile du copolymère attendu ; et si désiré
- Une étape c) d'élimination de l'huile de la solution de copolymère obtenue à l'étape b).

12. Solution de copolymère directement obtenue à l'issue de l'étape b) du procédé tel que défini à la revendication 10.

13. Utilisation du copolymère tel que défini à l'une quelconque des revendications 1 à 10, ou de la solution de polymère telle que définie à la revendication 12, comme agent modificateur de la rhéologie dans une composition topique cosmétique, dermopharmaceutique ou pharmaceutique.

14. Procédé pour modifier la rhéologie d'une composition topique cosmétique, dermopharmaceutique ou pharmaceutique comprenant une phase grasse, **caractérisé en ce que** l'on introduit dans ladite phase grasse une quantité efficace du copolymère tel que défini à l'une quelconques des revendications 1 à 10 ou de la solution de polymère telle que définie à la revendication 12.

15. composition topique cosmétique, dermopharmaceutique ou pharmaceutique et plus particulièrement une émulsion eau dans huile, **caractérisée en ce qu'**elle comprend pour 100% massique de 0,1% à 10% massique et plus particulièrement de 1% massique à 5% massique du copolymère tel que défini à l'une quelconques des revendications 1 à 10.

## Patentansprüche

1. Copolymer, **dadurch gekennzeichnet, dass** es auf 100 Mol-% Folgendes umfasst:
a) mehr als 70 Mol-% und bis zu 99,9 Mol-% hydrophobe Monomereinheiten (A), die von Stearylmethacrylat stammen; und
b) 0,1 Mol-% bis 30 Mol-% Monomereinheiten (B), die von mindestens einem Monomer einer Verbindung der Formel (I) stammen: 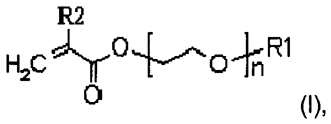 in der R1 für einen linearen oder verzweigten Alkylrest steht, der 12 bis 22 Kohlenstoffatome umfasst, R2 für ein Wasserstoffatom oder einen Methylrest steht und n eine ganze Zahl ist, die größer oder gleich 0 und kleiner oder gleich 30 ist, wobei es sich versteht, dass die Verbindung der Formel (I) nicht Stearylmethacrylat ist.

2. Copolymer nach Anspruch 1, wobei der Rest R1 in der Formel (I) für einen linearen Alkylrest steht, der aus Dodecyl- oder Docosanylresten ausgewählt ist.

3. Copolymer nach einem der Ansprüche 1 oder 2, wobei n in der Formel (I) größer als 0 ist und n insbesondere größer oder gleich vier und kleiner oder gleich fünfundzwanzig ist.

4. Copolymer nach Anspruch 3, **dadurch gekennzeichnet, dass** es sich um das Copolymer von Stearylmethacrylat und pentacosaethoxyliertem Behenylmethacrylat oder das Copolymer von Stearylmethacrylat und tetraethoxyliertem Laurylacrylat handelt.

5. Copolymer nach einem der Ansprüche 1 bis 4, wobei das Molverhältnis von (A):(B) zwischen 80:20 und 95:5 liegt.

6. Copolymer nach einem der Ansprüche 4 oder 5, **dadurch gekennzeichnet, dass** es sich um Folgendes handelt:
- Copolymer von Stearylmethacrylat und pentacosaethoxyliertem Behenylmethacrylat [Molverhältnis von (A):(B) = 95:5],
- Copolymer von Stearylmethacrylat und pentacosaethoxyliertem Behenylmethacrylat [Molverhältnis von (A):(B) = 90:10],
- Copolymer von Stearylmethacrylat und pentacosaethoxyliertem Behenylmethacrylat [Molverhältnis von (A):(B) = 85:15] oder:
- Copolymer von Stearylmethacrylat und pentacosaethoxyliertem Behenylmethacrylat [Molverhältnis von (A):(B) = 80:20].

7. Copolymer nach einem der Ansprüche 1 bis 6, **dadurch gekennzeichnet, dass** es außerdem auf 100 Mol-% 0,1 Mol-% bis 30 Mol-% Monomereinheiten (C) umfasst, die von mindestens einem neutralen Monomer stammen, das aus Acrylamid, Methacrylamid, N,N-Dimethylacrylamid, N,N-Diethylacrylamid, N-Isopropylacrylamid, (2-Hydroxyethyl)acrylat, (2,3-Dihydroxypropyl)acrylat, (2-Hydroxyethyl)methacrylat, (2,3-Dihydroxypropyl)methacrylat oder N-Vinylpyrrolidon ausgewählt ist.

8. Copolymer nach Anspruch 9, wobei die Monomereinheit (C) von N,N-Dimethylacrylamid oder N,N-Diethylacrylamid stammt.

9. Copolymer nach einem der Ansprüche 7 oder 8, das auf 100 Mol-% Folgendes umfasst:
a) 80 Mol-% bis 95 Mol-% hydrophobe Monomereinheiten (A);
b) 2,5 Mol-% bis 10 Mol-% Monomereinheiten (B) und
c) 2,5 Mol-% bis 10 Mol-% Monomereinheiten (C).

10. Copolymer nach einem der Ansprüche 8 oder 9, **dadurch gekennzeichnet, dass** es sich um Folgendes handelt:
- Terpolymer von Stearylmethacrylat, pentacosaethoxyliertem Behenylmethacrylat und N,N-Dimethylacrylamid [Molverhältnis von (A):(B):(C) = 87:5:8];
- Terpolymer von Stearylmethacrylat, pentacosaethoxyliertem Behenylmethacrylat und N,N-Dimethylacrylamid [Molverhältnis von (A):(B):(C) = 80:5:15];
- Terpolymer von Stearylmethacrylat, pentacosaethoxyliertem Behenylmethacrylat und N,N-Dimethylacrylamid [Molverhältnis von (A):(B):(C) = 85:10:5];
- Terpolymer von Stearylmethacrylat, pentacosaethoxyliertem Behenylmethacrylat und N,N-Dimethylacrylamid [Molverhältnis von (A):(B):(C) = 80:10:10];
- Terpolymer von Stearylmethacrylat, pentacosaethoxyliertem Behenylmethacrylat und N,N-Dimethylacrylamid [Molverhältnis von (A):(B):(C) = 90:5:5] oder
- Terpolymer von Stearylmethacrylat, pentacosaethoxyliertem Behenylmethacrylat und N,N-Dimethylacrylamid [Molverhältnis von (A):(B):(C) = 95:2,5:2,5)].

11. Verfahren zur Herstellung des Copolymers nach einem der Ansprüche 1 bis 10, wobei das Verfahren Folgendes umfasst:
- einen Schritt a) der Herstellung einer organischen Phase durch Mischen mindestens eines Öls mit dem Stearylmethacrylat, dem Monomer der Formel (I) und gegebenenfalls dem neutralen Monomer in den gewünschten Molanteilen;
- einen Schritt b) der Polymerisation, die durch Einbringen eines thermischen Initiators in das aus dem Schritt a) resultierende Gemisch eingeleitet wird, um eine Lösung des erwarteten Copolymers in dem Öl zu erhalten; und auf Wunsch
- einen Schritt c) der Entfernung des Öls aus der im Schritt b) erhaltenen Copolymerlösung.

12. Copolymerlösung, die direkt am Ende des Schritts b) des Verfahrens nach Anspruch 10 erhalten wird.

13. Verwendung des Copolymers nach einem der Ansprüche 1 bis 10 oder der Polymerlösung nach Anspruch 12 als Rheologiemodifikator in einer topischen kosmetischen, dermopharmazeutischen oder pharmazeutischen Zusammensetzung.

14. Verfahren zum Modifizieren der Rheologie einer topischen kosmetischen, dermopharmazeutischen oder pharmazeutischen Zusammensetzung, die eine Fettphase umfasst, **dadurch gekennzeichnet, dass** eine wirksame Menge des Copolymers nach einem der Ansprüche 1 bis 10 oder der Polymerlösung nach Anspruch 12 in die Fettphase eingebracht wird.

15. Topische kosmetische, dermopharmazeutische oder pharmazeutische Zusammensetzung und insbesondere Wasser-in-Öl-Emulsion, **dadurch gekennzeichnet, dass** sie auf 100 Masse-% 0,1 bis 10 Masse-% und insbesondere 1 Masse-% bis 5 Masse-% des Copolymers nach einem der Ansprüche 1 bis 10 umfasst.

## Claims

1. Copolymer, **characterised in that** it comprises, per 100 mol %:
a) more than 70 mol % and up to 99.9 mol % of hydrophobic monomer units (A) derived from stearyl methacrylate; and
b) from 0.1 mol % to 30 mol % of monomer units (B) derived from at least one monomer of a compound of formula (I): 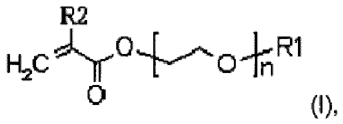 in which R1 represents a linear or branched alkyl radical comprising from 12 to 22 carbon atoms, R2 represents a hydrogen atom or a methyl radical and n is an integer greater than or equal to 0 and less than or equal to 30, it being understood that said compound of formula (I) is not stearyl methacrylate.

2. Copolymer according to claim 1, for which, in formula (I), the radical R1 represents a linear alkyl radical chosen from dodecyl or docosanyl radicals.

3. Copolymer according to either claim 1 or claim 2, for which, in formula (I), n is greater than zero and more particularly n is greater than or equal to four and less than or equal to twenty five.

4. Copolymer according to claim 3, **characterised in that** it is the copolymer of stearyl methacrylate and pentacosaethoxylated behenyl methacrylate or the copolymer of stearyl methacrylate and tetraethoxylated lauryl acrylate.

5. Copolymer according to any of claims 1 to 4, wherein the molar ratio (A)/(B) is between 80/20 and 95/5.

6. Copolymer according to either claim 4 or claim 5, **characterised in that** it is the:
- copolymer of stearyl methacrylate and pentacosaethoxylated behenyl methacrylate [molar ratio (A)/(B) = 95/5],
- copolymer of stearyl methacrylate and pentacosaethoxylated behenyl methacrylate [molar ratio (A)/(B) = 90/10],
- copolymer of stearyl methacrylate and pentacosaethoxylated behenyl methacrylate [molar ratio (A)/(B) = 85/15], or the
- copolymer of stearyl methacrylate and pentacosaethoxylated behenyl methacrylate [molar ratio (A)/(B) = 80/20].

7. Copolymer according to any of claims 1 to 6, **characterised in that** it also comprises, per 100 mol %, from 0.1 mol % to 30 mol % of monomer units (C) derived from at least one neutral monomer selected from acrylamide, methacrylamide, N,N-dimethylacrylamide, N,N-diethylacrylamide, N-isopropylacrylamide, 2-hydroxyethyl acrylate, 2,3-dihydroxypropyl acrylate, 2-hydroxyethyl methacrylate, 2,3-dihydroxypropyl methacrylate or N-vinylpyrrolidone.

8. Copolymer according to claim 9, wherein said monomer unit (C) is derived from N,N-dimethylacrylamide or from N,N-diethylacrylamide.

9. Copolymer according to either claim 7 or claim 8, comprising, per 100 mol %:
a) from 80 mol % to 95 mol % of hydrophobic monomer units (A);
b) from 2.5 mol % to 10 mol % of monomer units (B); and
c) from 2.5 mol % to 10 mol % of monomer units (C).

10. Copolymer according to either claim 8 or claim 9, **characterised in that** it is the:
- terpolymer of stearyl methacrylate, pentacosaethoxylated behenyl methacrylate and N,N-dimethylacrylamide [molar ratio (A)/(B)/(C) = 87/5/8];
- terpolymer of stearyl methacrylate, pentacosaethoxylated behenyl methacrylate and N,N-dimethylacrylamide [molar ratio (A)/(B)/(C) = 80/5/15];
- terpolymer of stearyl methacrylate, pentacosaethoxylated behenyl methacrylate and N,N-dimethylacrylamide [molar ratio (A)/(B)/(C) = 85/10/5];
- terpolymer of stearyl methacrylate, pentacosaethoxylated behenyl methacrylate and N,N-dimethylacrylamide [molar ratio (A)/(B)/(C) = 80/10/10];
- terpolymer of stearyl methacrylate, pentacosaethoxylated behenyl methacrylate and N,N-dimethylacrylamide [molar ratio (A)/(B)/(C) = 90/5/5]; or the
- terpolymer of stearyl methacrylate, pentacosaethoxylated behenyl methacrylate and N,N-dimethylacrylamide [molar ratio (A)/(B)/(C) = 95/2.5/2.5].

11. Process for preparing the copolymer according to any of claims 1 to 10, comprising:
- a step a) of preparing an organic phase by mixing, in the desired molar proportions, at least one oil with stearyl methacrylate, the monomer of formula (I) and, where appropriate, the neutral monomer;
- a step b) of polymerisation initiated by introducing a thermal initiator into the mixture resulting from step a) to obtain a solution in oil of the expected copolymer; and, if desired
- a step c) of removing the oil from the copolymer solution obtained in step b).

12. Copolymer solution directly obtained after step b) of the process according to claim 10.

13. Use of the copolymer according to any of claims 1 to 10, or of the polymer solution according to claim 12, as a rheology modifier in a cosmetic, dermopharmaceutical or pharmaceutical topical composition.

14. Process for modifying the rheology of a cosmetic, dermopharmaceutical or pharmaceutical topical composition comprising a fatty phase, **characterised in that** an effective amount of the copolymer according to any of claims 1 to 10 or of the polymer solution according to claim 12 is introduced into said fatty phase.

15. Cosmetic, dermopharmaceutical or pharmaceutical topical composition, more particularly a water-in-oil emulsion, **characterised in that** it comprises, per 100 % by weight, from 0.1 % by weight to 10 % by weight, more particularly from 1 % by weight to 5 % by weight, of the copolymer according to any of claims 1 to 10.
